# EUROPEAN PATENT APPLICATION

(11) **EP 0 537 116 A1**
(43) Date of publication of application: **14.04.1993**
(21) Application number: 92830149.8
(22) Date of filing: 25.03.1992
(51) Int. Cl.: A61B 17/32, A61F 9/00

(54) **Double duct surgical instrument**

(30) Priority: 11.10.1991 IT RM910775
(71) Applicant: Caponi, Mauro, I-00148 Roma (IT)
(72) Inventor: Caponi, Mauro, I-00148 Roma (IT)
(74) Representative: Iannone, Carlo Luigi

(57) **Abstract**

The invention relates to a surgical instrument (1) comprising an outer hollow duct (2), having a closed front end and a first port (4) near said front end, an inner hollow duct (3), slidingly arranged in said outer duch (2), provided with a front cutting blade (11) and internally connected with vacuum means, as well as a hollow interspace (6) between said inner (3) and outer (2) ducts arranged for a fluid flow; characterized in that said inner duct (3) has a constant cross-section, in that said outer duct (2) has at its front end a portion with a cross-section reduced to such an extent as to enable only the inner duct (3) to slide therein, whereby said interspace (6) is minimized, and in that in said outer duct (2), near the reduced cross-section portion (5) but on its side having a larger cross-section, at least a second port (8) is provided such that, during a surgical operation, the fluid outflows therefrom toward the human body and re-enters though said first port (4) under action of said vacuum means.

## Description

This invention relates to a double duct surgical instrument, particularly designed for ablative operations in internal cavities of the body.

More detailedly, this invention relates to an improved surgical instrument that enables surgical operations in particular diskectomies to be better performed.

The instrument of the type disclosed in this patent application is particularly used in surgical operations performed without cutting actions upon the patient, but simply introducing the tube into the patient's body.

Operations of this kind are now performed in an ever increasing number of cases, such as for instance in ocular surgery, in herniated disk operations (diskectomy), etc..

Surgical instruments according to the state of the art comprise an outer stationary duct and an inner duct, coaxial to the outer duct, having at its front end a cutting blade and internally hollow so as to be connected with a vacuum source.

Between the inner and the outer ducts an interspace is formed through which a flow of water is directed.

The inner duct has an enlarged cross-section near its cutting blade, so as to establish a point of minimum distance with respect to the inner surface of the outer duct. A small port is provided in the inner duct, near its cutting blade, so as to enable the water to flow from said interspace into said inner duct.

At its front end, the outer duct has a port by which one is enabled to operate upon the concerned portion of the human body.

In fact, when the duct is inserted into the concerned portion of the human body, for instance into the disk, when a diskectomy operation is to be performed, the disk tissue penetrates into the outer duct through said port and is cut by the cutting blade of the inner duct.

The cut away portions of the disk tissue are drawn through the internal lumen of the inner duct and the water flowing into it acts as a carrier to aid the entrainment thereof.

From a construction view point, providing said port in the inner duct and enlarging it at the end section entail noticeable problems.

In fact, in many cases, water is employed as a carrier for entrainment of pharmaceutical products aimed at preventing infections to develop during the surgical operation.

Anyway, since the water is drawn into the inner duct and since it is directly passes through said port, the amount of water contacting the human body is effectively small, and consequently also the disinfecting action is scarce.

It is the main object of this invention to provide an improved surgical instrument that is structurally simpler than the already known instruments.

A further object of this invention is to provide an instrument of the above set forth kind that enables a circulation of the water employed for removing the particles cut during the intracorporeal surgical operation, so that the disinfecting action of the pharmaceutical preparations dissolved therein is optimum.

It is, therefore, specific subject-matter of this invention a surgical instrument comprising an outer hollow duct having a closed front end and a first port near said front end, an inner hollow duct, slidingly arranged in said outer duct, provided with a front cutting blade and internally connected with a vacuum means, as well as a hollow interspace between said inner and outer ducts arranged for a fluid flow; characterized in that said inner duct has a constant cross-section, in that said outer duct has at its front end a portion with a cross-section reduced to such an extent as to enable only the inner duct to slide therein, whereby said interspace is minimized, and in that in said outer duct, near the reduced cross-section portion but on its side having a larger cross-section, at least a second port is provided such that, during a surgical operation, the fluid outflows therefrom toward the human body and re-enters through said first port under action of said vacuum means.

In particular, according to the invention, only a single second port is provided near the reduced cross-section portion of the outer duct.

Preferably, said fluid flowing through the interspace is water containing, if desired, pharmaceutical preparations.

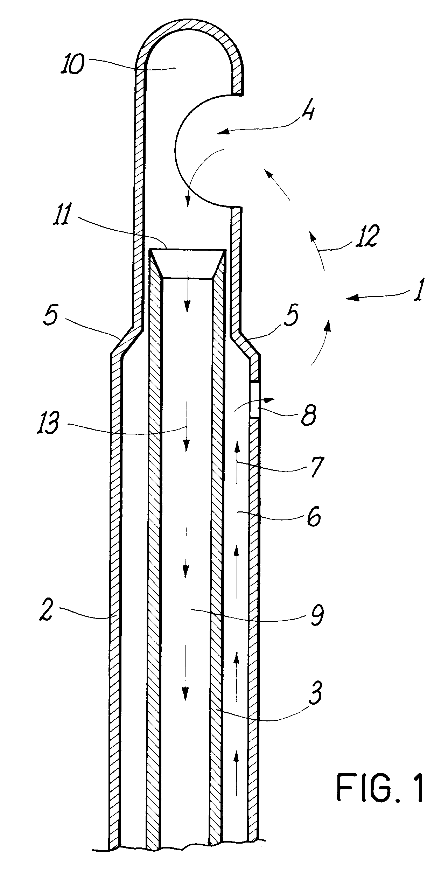

This invention will be now described by way of illustration not by way of limitation, with reference to a preferred embodiment thereof as shown in Fig. 1 that is a longitudinal cross-section view of a surgical instrument according to this invention.

The instrument 1 according to the invention consists of a hollow outer duct 2 and of a similar hollow inner duct 3, slidingly arranged in said outer duct 2.

The outer duct 2 is frontally closed and has a port 4 near its front end.

A reduced cross-section portion 5 is provided on the outer duct 2, such that the interspace 6 formed between the outer duct 2 and the inner duct 3 is restricted in order to form an interspace or air gap just sufficient to permit the inner duct 3 to slide therein.

Water flows in said interspace 6 according to the directional arrows 7.

Near the reduced cross-section portion 5, a port 8 is provided on the outer duct 2 so as to permit a water outflow therethrough, no direct connection existing between the interspace 6 and the lumen 9 of the inner duct 3.

During the diskectomy operation, the instrument 1 according to the invention is introduced into the disk of the patient. The concerned disk, therefore, penetrates through port 4 into the front cavity 10 of the outer duct 2 and is cut by the cutting blade 11 provided at the front end of the inner duct 3, which is rectilinearly reciprocally moved.

The fragments of the disk as cut are drawn away through lumen 9 connected to vacuum means (not shown).

The water having a disinfectant pharmaceutical product added thereto outflows toward the body of the patient through port 8 and re-enters according to the arrows 12, under action of the vacuum means, through port 4, whereby the removed fragments are entrained according to arrows 13.

During its intracorporeal run, said water enables the tissue upon which the surgical operation is carried out to be disinfected by means of the pharmaceutical product dissolved therein.

This invention has been described under specific reference to some preferred embodiments, but it should be understood that variations and/or changes can be made thereto by those skilled in the art without departing from its scope.

## Claims

1. A surgical instrument comprising an outer hollow duct having a closed front end and a first port near said front end, an inner hollow duct, slidingly arranged in said outer duct, provided with a front cutting blade and internally connected with vacuum means, as well as a hollow interspace between said inner and outer ducts arranged for a fluid flow; characterized in that said inner duct has a constant cross-section, in that said outer duct has at its front end a portion with a cross-section reduced to such extent as to only enable the inner duct to slide therein, whereby said interspace is minimized, and in that in said outer duct, near the reduced cross-section portion but on its side having a larger cross-section, at least a second port in provided such that, during a surgical operation, the fluid outflows therefrom toward the human body and re-enters through said first port under action of said vacuum means.

2. A surgical instrument according to claim 1, characterized in that only a single second port is provided near the reduced cross-section portion of the outer duct.

3. A surgical instrument according to any preceding claim, characterized in that said fluid flowing in the interspace is water containing pharmaceutical preparations dissolved therein.

4. A surgical instrument according to any preceding claim, substantially as shown and disclosed.
